# EUROPÄISCHE PATENTSCHRIFT

(11) **EP 1 682 146 B1**
(45) Veröffentlichungstag und Bekanntmachung des Hinweises auf die Patenterteilung: **09.01.2008**
(21) Anmeldenummer: 04704998.6
(22) Anmeldetag: 24.01.2004
(51) Int. Cl.: A61K 31/506, A61K 8/49, A61Q 3/02, A61P 31/10

(54) **ANTIMYKOTISCH WIRKSAME NAGELLACKFORMULIERUNGEN MIT SUBSTITUIERTEN 2-AMINOTHIAZOLEN ALS WIRKSTOFF**
ANTIMYCOTIC NAIL POLISH FORMULATIONS COMPRISING SUBSTITUTED 2-AMINOTHIAZOLES AS AN ACTIVE SUBSTANCE
FORMULATIONS DE VERNIS A ONGLES ANTIMYCOSIQUES CONTENANT DES 2-AMINOTHIAZOLES SUBSTITUES COMME AGENTS ACTIFS

(30) Priorität: 11.09.2003 DE 10341944
(43) Veröffentlichungstag der Anmeldung: 26.07.2006
(73) Patentinhaber: York Pharma PLC, Pirton, Hitchin, Herts SG5 3QN (GB)
(72) Erfinder: LANG-FUGMANN, Susanne, 40878 Ratingen (DE)
(74) Vertreter: Elend, Almut Susanne
(86) Internationale Anmeldenummer: PCT/EP2004/000597
(87) Internationale Veröffentlichungsnummer: WO 2005/034956

(56) Entgegenhaltungen:
- EP-A- 0 365 915
- WO-A-00/38732
- WO-A-98/30212
- WO-A-99/39680
- ZIEGELBAUER KARL: "A dual labelling method for measuring uptake of low molecular weight compounds into the pathogenic yeast Candida albicans" MEDICAL MYCOLOGY, Bd. 36, Nr. 5, Oktober 1998 (1998-10), Seiten 323-330, XP009031973 ISSN: 1369-3786
- FLECKMAN P: "Onychomycosis: Diagnosis and topical therapy" DERMATOLOGIC THERAPY 2002 UNITED STATES, Bd. 15, Nr. 2, 2002, Seiten 71-77, XP002285938 ISSN: 1396-0296
- COHEN P R ET AL: "TOPICAL AND SURGICAL TREATMENT OF ONYCHOMYCOSIS" JOURNAL OF THE AMERICAN ACADEMY OF DERMATOLOGY, C.V. MOSBY, ST. LOUIS, MO, US, Bd. 31, Nr. 3, 1994, Seiten S74-S77, XP008012712 ISSN: 0190-9622

## Beschreibung

### Hintergrund der Erfindung

### 1. Gebiet der Erfindung

Die Erfindung betrifft antimykotische Nagellackformulierungen zur Behandung von Onychomykosen sowie anderer, durch Pilzinfektionen verursachten Erkrankungen von Finger- oder Zehnägeln. Insbesondere betrifft die Erfindung antimykotisch wirksame Nagellackformulierungen, die auf Nägel aufgetragen werden und die einen Überzug bilden, aus dem das antimykotisch wirksame Mittel freigesetzt wird und in den Nagel eindringen kann. Mit dieser Methode können Nägel von Mensch und Tier behandelt werden, um Pilzinfektionen zu behandeln oder diesen vorzubeugen.

### 2. Stand der Technik

Pilzinfektionen von Nägeln, im allgemeinen als Onychomykosen bezeichnet, werden am häufigsten verursacht durch Dermatophyten, können aber auch durch Schimmelpilze und Hefepilze verursacht werden. Mischinfektionen treten ebenfalls auf. Onychomykosen schließen daher durch Dermatophyten verursachte Infektionen der Nagelplatte und von Nägeln ebenso ein wie durch andere Pilzarten, einschließlich Schimmelpilze und Hefen verursachte Erkrankungen. Darüber hinaus können Onychomykosen z.B. als Reservoir für Dermatophyten dienen und somit zu Behandlungsversagen bei Tinea pedis und zu Reinfektionen von Tinea pedis führen.

Am häufigsten werden Onychomykosen durch *Trichophyton rubrum, T. mentagrophytes* und *Epidermophyton floccusum* verursacht. Onychomykosen, die nicht durch Dermatophyten verursacht werden, werden normalerweise durch Candida Spezies verursacht.

Onychomykosen verursachen Verdickungen und Verfärbungen des Nagels und können sogar den Verlust oder die Zerstörung des Nagels zur Folge haben, darüber hinaus können sie die Ursache sein für Schmerzen, ungenügende Durchblutung, Problemen beim Gehen und für andere unerwünschte Erscheinungen.

Onychomykosen wurden in der Vergangenheit unter anderem dadurch behandelt, dass der befallene Teil des Nagels oder der ganze Nagel entfernt wurde. Diese Art der Behandlung kann jedoch zu einer dauerhaften Beschädigung des Nagels führen. Auch kann der nachwachsende Nagel in einer missgebildeten Form nachwachsen. Darüber hinaus gibt es keine Garantie dafür, dass die Onychomykose durch eine Nagelentfernung komplett geheilt werden kann.

Anstatt durch eine Nagelentfernung kann eine Onychomykose auch durch die Anwendung von verschiedenen Antimykotika behandelt werden. Dabei können die Antimykotika beispielsweise oral verabreicht werden. Bei dieser Form der Behandlung erfolgt jedoch eine Belastung des gesamten Körpers und nur eine geringe Menge der antimykotisch wirksamen Substanz erreicht den Nagel über die Nagelmatrix. Die orale Behandlung hat weiterhin den Nachteil, dass eine solche Behandlung eine Behandlungszeit von mindestens 12 Wochen für Zehnägel und etwa 6 - 8 Wochen für Fingernägel benötigt. Eine solch lange Behandlungszeit verursacht hohe Kosten für die Behandlung. Auch wird die Patientencompliance deutlich reduziert. Weiterhin erhöht die orale Behandlung das Risiko von Nebenwirkungen, wie z. B. Reizungen des Magen-Darm-Traktes, Übelkeit, unerwünschte Wechselwirkungen mit anderen Arzneimitteln, wirkstoffinduzierte Hautauschläge und andere unerwünschte Nebeneffekte. Darüber hinaus erschweren beispielweise variable Absorptionsraten und Metabolismusgeschwindigkeiten die orale Behandlung von Onychomykosen.

Eine weitere Methode zur Behandlung von Onychomykosen besteht darin, eine pharmazeutische Formulierung, die einen antimykotischen Wirkstoff enthält, topisch anzuwenden. So ist bekannt, Onychomykosen durch Nagellackformulierungen, in denen ein antimykotischer Wirkstoff enthalten ist, zu behandeln.

Nagellackformulierungen für die Behandlung von Onychomykosen und ähnlicher Pilzinfektionen von Nägeln, und zwar sowohl von Finger- als auch von Zehnägeln von Menschen, aber auch von Tieren, sind bekannt.

Einige Beispiele sind in der Patentliteratur beschrieben. Erwähnt werden können in diesem Zusammenhang z. B.:
US Pat. 4,957,730 (1-Hydroxy-2-pyridon in einem wasserunlöslichen Filmbildner);
US Pat. 5,120,530 (Amorolfin in einem quaternären Ammoniumacrylat Copolymeren);
US Pat. 5,264,206 (Tioconazol, Econazol, Oxiconazol, Miconazol, Tolnaftat, Naftifin- Hydrochlorid, in wasserunlöslichen Filmbildnern);
US Pat. 5,346,692 (mit Harnstoff und Dibutylphthalat als Weichmacher);
US Pat. 5,487,776 (Griseofulvin in einer kolloidalen Suspension).
Weitere Patente mit anderen Beispielen sind z. B. 4,636,520; 5,002,938; 5,110,809; 5,219,877; 5,391,367; 5,464,610; 5,696,105; 5,814305; 6,224,887; 6,495,124 und WO Nr. 01160325.

Die Effektivität von Nagellacken, die als antimykotisches Mittel Amorolfin enthalten, wird beschrieben von Jean-Paul L. Marty, J. of the European Academy of Dermatology and Venereology, 4 (Suppl. 1), 17-21 (1995). Vom Autor wird beschrieben, dass die filmbildende Lösung des Nagellackes prinzipiell aus dem Wirkstoff und einem flüchtigen Lösungsmittel besteht (Ethanol, Ethyl-, Butyl-, Methylacetat, Methylenchlorid, Methylethylketon, Isopropanol) sowie einem wasserunlöslichen Polymer (Methacrylsäure Copolymer, Vinylpolymere), die einen zusammenhängenden Film bilden, nachdem das Lösungsmittel verdampft ist. Weichmacher (Triacetin, Dibutylphthalat) verleihen dem Nagellack eine ausreichende mechanische Flexibilität, um ein Abblättern oder einen Abrieb des Lackes zu vermeiden.

Eine Zusammenfassung der Struktur und der Charakteristika des Nagels sowie eine Diskussion der Durchlässigkeit der Nagelplatte gegenüber verschiedenen Agenzien sowie der Durchlässigkeit gegenüber Alkoholen wird durch K. A. Walter und G. L. Flynn gegeben: "Permeability characteristics of the human nail plate", Int. J. of Cosmetic Science, 5, 231 to 246 (1983).

In neuerer Zeit wurden antimykotisch wirkende Nagellackformulierungen entwickelt, die 2-n-Nonyl-1,3-dioxolan oder verwandte Dioxane and Acetalverbindungen als Haut- und Nagelpenetrationsbeschleuniger enthalten. Auch wurde gefunden, dass diese Penetrationsbeschleuniger als Weichmacher für filmbildende Polymere fungieren. Daher können Nagellackformulierungen, die Dioxolane, Dioxane und Acetale als Penetrationsbeschleuniger enthalten, ohne zusätzliche Weichmacher hergestellt werden. Dabei bleiben die Lacke aus diesen Formulierungen transparent, hart und wasserbeständig, sie haften gut auf Nägeln und geben darüber hinaus das Antimykotikum in ausreichender Menge in den Nagel ab. Derartige antimykotisch wirksame Nagellackformulierungen werden beansprucht in WO 99/39680.

Aus den US-Patenten 5,914,322 und 5,962,433 ist bekannt, dass Hyaluronsäure als Penetrationsbeschleuniger verwendet werden kann. Darüber hinaus werden wirksame antimykotische Nagellackformulierungen beschrieben, in denen Hyaluronatlyasen, die aus unterschiedlichen mikrobiellen Quellen gewonnen werden können, als Penetrationsbeschleuniger verwendet werden (WO 00/38732).

Die in den vorbeschriebenen Nagellackformulierungen eingesetzten Antimykotika haben jedoch alle einen unzureichenden Effekt zur Behandlung von Onychomykosen, da die verwendeten Wirkstoffe keine ausreichende Wirkung gegenüber den die Onychomykosen verursachenden Erregern haben. Wegen der fungiziden Wirkungen gegen wachsende und ruhende Keime ermöglichen die antimykotisch wirksamen substituierten 2-Aminothiazole eine wirkungsvolle und erfolgreiche Behandlung von Onychomykosen, wenn sie in Nagellackformulierungen zur Anwendung kommen. Derartige Nagellackformulierungen mit substituierten 2-Aminothiazolen als Wirksubstanz sind Gegenstand der vorliegenden Erfindung.

Gegenstand der vorliegenden Erfindung ist eine Zusammensetzung aus (A) einer pharmazeutisch wirksamen Verbindung aus der Verbindungsklasse der antimykotisch wirksamen substituierten 2-Aminothiazole und (B) einer Nagellackformulierung dieser Verbindung, die einen Pentrationsbeschleuniger enthält.

Die pharmazeutisch wirksamen Verbindungen der antimykotisch wirksamen substituierten 2-Aminothiazole werden in den US-Patenten 4,956,370 und 5,104,879 beschrieben.

Die substituierten 2-Aminothiazole haben die allgemeine Formel (I) 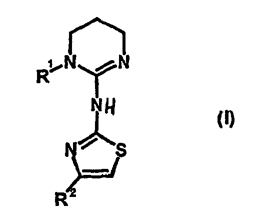 in der
- R¹: Wasserstoff oder Alkyl bedeuten und
- R²: einen Substituenten der Formel 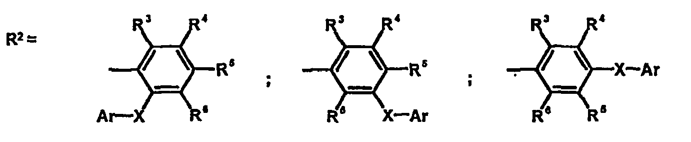
bedeutet,
in dem R³ , R⁴, R⁵ and R⁶ unabhängig voneinander Wasserstoff, Halogen, Nitro, Alkyl, Alkoxy, Alkoxycarbonyl, Dialkylamino, Alkylthio, Alkylsulphinyl, Alkylsulphonyl, Halogenalkyl, Halogenalkoxy, Halogenalkylthio, Halogenalkylsulphinyl oder Halogenalkylsulphonyl bedeuten,
X Sauerstoff, Schwefel, Sulphinyl oder Sulphonyl bedeuten und
Ar einen ggf. substituierten Arylrest bedeutet
und deren physiologisch verträgliche Säureadditionssalze.

Die Verbindungen der Formel (I) stehen im Gleichgewicht mit den tautomeren Verbindungen der Formeln (la) und (Ib) 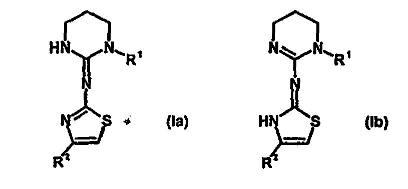 in denen R¹ and R² in jedem Falle die o. a. Bedeutungen haben.

Besonders wirksam ist die Verbindung der Formel (I) bzw. (1a) oder (1b) mit
R¹ = H und R² =

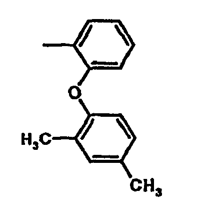

Dieser Wirkstoff heißt Abafungin (gemäß British Pharmacopoeia).

Die Nagellacksysteme, in denen Abafungin angewandt werden können, entsprechen vorbeschriebenen Systemen (s. z. B. Poucher's Perfumes, Cosmetics and Soaps, Hrsg. H. Butler, Kluwer Academic Publishers, Dordrecht, Boston, London 2000, 330 - 343; Sudaxshina Murdan, Int. J. Pharmaceutics 236 (2002) 1 - 26), den weiter oben aufgeführten Patenten sowie der Literatur, die in diesem Publikationen und Patenten zitiert wird).

Die Nagellacksysteme sollten einfach anzuwenden sein, schnell härten, schnell trocknen, wasserfest sein, gut haften, elastisch sein und widerstandsfähig gegen Abblätterung und Abrieb sein. Die zur Verwendung kommenden Einsatzmaterialien dürfen nicht toxisch sein und müssen dermatologisch unschädlich sein. Die Hauptbestandteile derartiger Nagellacksysteme sind ein Filmbildner, ein Polymer, ein Weichmacher und Lösungsmittel. Weiterhin können Haut- bzw. Nagelpenetrationsbeschleuniger Bestandteil der Nagellackformulierung sein. Wie oben erwähnt, wurde in einigen Erfindungen beschrieben, dass es Beschleuniger gibt, die gleichzeitig als Weichmacher für das filmbildende Polymer wirken.

Der Gegenstand der vorliegenden Erfindung bedeutet einen wichtigen Fortschritt gegenüber dem Stand der Technik, da mit der erfindungsgemäßen Nagellackformulierung, die einen Wirkstoff aus der Verbindungsklasse der antimykotisch wirksamen substituierten 2-Aminothiazole enthalten, Onychomykosen und andere Pilzinfektionen sowie bakterielle Infektionen und Entzündungen, einschließlich Psoriasis, erfolgreich behandelt werden können.

### Beschreibung der Erfindung

Die pharmazeutische Formulierung der vorliegenden Erfindung enthält eine pharmazeutisch wirksame Menge einer antimykotisch wirksamen Verbindung aus der Substanzklasse der substituierten 2-Aminothiazole (US-Patente 4,956,370 und 5,104,879). Insbesondere enthält die wirksame pharmazeutische Formulierung Abafungin als Wirkstoff. Diese Nagellackformulierungen mit substituierten 2-Aminothiazolen als Wirkstoff können sowohl als Therapeutikum als auch als Prophylaktikum eingesetzt werden. Die substituierten 2-Aminothiazole können auch in Kombination mit einem entzündungshemmenden Mittel und/oder einem anderen antimikrobiellen Wirkstoff, wie z. B. einem antibakteriellen oder antiviralen Wirkstoff, angewandt werden.

Die Konzentrationen des substituierten 2-Aminothiazols, insbesondere des Abafungins, sollten die maximale Menge nicht überschreiten, die in der pharmazeutischen Formulierung gelöst bleiben. Die meisten Anwendungsformulierungen enthalten die pharmazeutisch wirksame Substanz aus der Klasse der substituierten 2-Amino-thiazole in einer Menge von 0,05 % bis 20 % (die Angaben erfolgen jeweils in Gewichtsprozenten, bezogen auf das Gesamtgewicht der pharmazeutischen Formulierung). Bevorzugt liegt die Menge der Verbindung bei 1 % bis 12 % (Gewichtsprozente), besonders bevorzugt liegt die Menge zwischen 1 % und 10 % (Gewichtsprozente), ganz besonders bevorzugt liegt die Menge zwischen 1,5 % und 8 % (Gewichtsprozente).

Die pharmazeutische Formulierung kann weiterhin einen membranverträglichen Penetrationsbeschleuniger enthalten, der in der Lage ist, die Menge des pharmazeutischen Wirkstoffs, der durch den Nagel und/oder eine Membran, das bedeutet eine Gewebsschicht des Körpergewebes, z. B. der Haut, tritt, zu beschleunigen. Der Begriff "membranverträglicher Penetrationsbeschleuniger" bezeichnet in diesem Zusammenhang eine Verbindung, die die Penetration des pharmazeutischen Wirkstoffs in den Nagel oder die Haut ohne Beschädigungen erleichtert.

Es ist bekannt, dass es Penetrationsbeschleuniger gibt, die über Mechanismen wie z.B. Hydrolyse, Keratolyse, Denaturierung oder weitere Mechanismen wirken, die den Nagel und/oder die Haut beschädigen. Beispiele solcher Penetrationsbeschleuniger sind Harnstoff, Sulfhydrylgruppen enthaltende Aminosäuren, Alkylsulfoxide und verwandte Verbindungen. Derartige Beschleuniger wirken durch den Verlust der Struktur des Nagels und der Haut, so dass der pharmazeutische Wirkstoff unerwünschterweise in tiefer gelegene Hautschichten eindringen oder die Hautschichten ganz durchdringen kann und somit nicht erwünschte Nebenwirkungen erzeugen kann.

Der membranverträgliche Penetrationsbeschleuniger muss sicher bei der Behandlung von Infektionen von Nagel und Haut angewandt werden können, ohne dass dabei Beschädigungen von Nagel oder Haut auftreten können.

Im wesentlichen können alle membrankompatiblen Penetrationsbeschleuniger oder Mischungen von Penetrationsbeschleunigern angewandt werden. Bevorzugt eingesetzte membrankompatible Penetrationsbeschleuniger sind lipophil, so dass sie es erlauben, Membranen permeabler zu machen, indem sie sich in die Membranen einlagern. Beispiele für derartige lipophile Penetrationsbeschleuniger sind Alkylester, wie z. B. Isopropylmyristat und Myristylmyristat sowie macrocyclische Beschleuniger.

Die membranverträglichen Penetrationsbeschleuniger werden in einer Menge in der pharmazeutischen Formulierung eingesetzt, die eine Penetration des pharmazeutischen Wirkstoffs in Nagel und Haut beschleunigt. Die wirksame Menge ist dem Fachmann bekannt bzw. kann durch den Fachmann leicht und einfach bestimmt werden. In der Regel liegt die Menge des verwendeten membranverträglichen Penetrationsbeschleunigers zwischen 1 % und 25 % (Gewichtsprozente, bezogen auf das Gesamtgewicht der pharmazeutischen Formulierung).

Die pharmazeutisch wirksame Formulierung kann weiterhin ein polymeres filmbildendes Agenz oder Mischungen von filmbildenden Agenzien enthalten. Im wesentlichen kann jedes Polymer genutzt werden, das einen Film bilden kann, aus dem der pharmazeutische Wirkstoff in den Nagel und auch das Nagelbett oder die Membran, z.B. die Haut, abgegeben werden kann. Z. B. können okklusive oder halb-okklusive Polymere, die für transdermale Arzneimittelanwendungen bekannt sind, angewandt werden. Da das primäre Ziel die Arzneimittelabgabe ist, können sogar solche Polymere verwendet werden, die ansonsten für eine kosmetische Anwendung aufgrund nicht idealer kosmetischer Eigenschaften nicht in Frage kommen.

Beispiele für mögliche filmbildende Polymere sind Polymere der Acrylsäure, Acrylsäureester und Copolymere von diesen; Polymere der Methacrylsäure, Methacrylsäureester und Copolymere von diesen; Polymere des Vinylacetates und Copolymerer von diesem mit Acrylsäure und Acrylsäureester; Copolymere von Methylvinylether mit Maleinsäure, Maleinsäurealkylester sowie Kombinationen von diesen; Copolymere von Vinylpyrrolidon mit Styro!; Poly(vinylbutyrat); polymere Cellulosederivate, wie Celluloseacetatephthalat, Cellulosebutylacetat, Celluloseacetylpropionat, Nitrocellulose, Cellulosesulfat, Ethylcellulose und Celluloseacetat; Terpolymere von Vinylacetat mit Butylmaleat und lsobornylacetat; Terpolymere von Vinylcaprolactam mit Vinylpyrrolidon und Dimethylaminoethylmethacrylat. Die filmbildenden Agenzien können in fester Form, z. B. in Pulverform, angewandt werden. Darüber hinaus kann die Anwendung in einer Latexform erfolgen.

Bevorzugt werden quartäre Ammoniumgruppen enthaltende Acrylsäureester, Methacrylsäureester Copolymere, bekannt als Ammoniomethacrylat Copolymere, wie z. B. Ethylacrylat-[2-(Methacryloyloxy)ethyl] trimethylammoniumchlorid-Methylmethacrylat Copolymer und substituierte Copolymere von alkylierten Poly(vinylpyrrolidonen) als filmbildende Agenzien verwendet. Diese polymeren filmbildenden Agenzien werden bevorzugt, da sie überlegene Adhäsionseigenschaften aufweisen, wasserbeständig sind und eine besondere Härte aufweisen. Besonders bevorzugt werden polymere filmbildende Agenzien eingesetzt, die von Zulassungsbehörden registriert worden sind zum Zwecke ihrer pharmazeutischen Verwendung und die auch, aber nicht-allein, in der Pharmacopoeia Europas und der der Vereinigten Staaten von Amerika sowie der japanischen Pharmaceutical Excipients Compendia aufgelistet sind.

Verwendbare filmbildende Polymere sind käuflich, wie z. B. die Acrylsäure Copolmeren der National Starch Co. unter dem Handelsnamen DEMACRYL®, z. B. DERMACRYL 79®, DERMACRYL LT®, die Amin- oder quartäre Ammoniumgruppen enthaltenden Acrylsäure Copolymere von Röhm unter dem Handelsnamen EUDRAGIT®, z. B. EUDRAGITs E®, RS®,RL®, den Methylvinylether Copolymeren der ISP Corp. unter dem Handelsnamen GANTREZ®, z. B. GANTREZ ES-3351®, GANTREZ ES-425®, ES-435® sowie den quartären Ammoniumacrylsäure Copolymeren der National Starch Co. unter dem Handelsnamen AMPHOMER®, z. B. AMPHOMER LV-71®.

Das polymere filmbildende Agenz wird in einer Menge eingesetzt, die es ermöglicht, einen adhärenten polymeren Film auf dem Nagel oder der Membran, d.h. der Haut, zu bilden. Die Mengen können für jede spezifische Anwendung durch den Fachmann leicht bestimmt werden.

In der Regel werden bei den meisten Anwendungen die polymeren filmbildenden Agenzien in einer Menge von 0,1 % bis 35 %, bevorzugt von 5 % bis 35 % und typischerweise und damit am bevorzugtesten in einer Menge von 10 % bis 25 % (alle Angaben in Gewichtsprozent, bezogen auf das Gesamtgewicht der Formulierung) angewandt.

Die pharmazeutische Formulierung kann weiterhin ein Lösungsmittel enthalten, um feste Inhaltsstoffe zu lösen. Im wesentlichen kann jedes Lösungsmittel oder jede Kombination von Lösungsmitteln eingesetzt werden. Beispiele solcher Lösungsmittel sind Alkohole, Ester, Ether, aromatische Kohlenwasserstoffe, Aldehyde, Ketone, Mono-, Di- und Triglyceride und ähnliche.

Die in der vorliegenden Erfindung eingesetztbaren Lösungsmittel sind nicht beschränkt, sollten aber aus der Gruppe der normalerweise physiologisch sicheren organischen Lösungsmittel für Lackformulierungen ausgewählt werden. Wichtig ist, dass die pharmazeutische Wirkung des eingesetzten Wirkstoffes nicht beeinflusst wird, dass der Lack leicht anwendbar ist und dass das Lösungsmittel leicht flüchtig ist, um akzeptable Trocknungszeiten einhalten zu können.

In der Regel werden die im folgenden aufgeführten Lösungsmittel eingesetzt: Ethanol, Ethylacetat, Butylacetat, Isopropanol, Aceton, Methylethylketon, Triacetin, Tripropionin, Diethylenglycolmonoethylether und Isopropylacetat sowie Mischungen von zweien oder mehreren der angegebenen Lösungsmittel.

Ethanol, Ethylacetat, Butylacetat, Isopropanol, Methylethylketon und Aceton sind besonders bevorzugte Lösungsmittel, da sie nach der Anwendung schnell verdampfen und der Lack schnell trocknet.

Das Lösungsmittel muss in der pharmazeutischen Formulierung in einer Menge vorhanden sein, dass alle Inhaltsstoffe gelöst sind, ohne dass aber die Trocknnngszeiten unbefriedigend lang und die Eigenschaften des Lacks negativ beeinflusst werden. In der Regel wird das Lösungsmittel in einer Menge von 30 % bis 80 % (Gewichtsprozente), besonders bevorzugt in einer Menge von 40 % bis 70 % (Gewichtsprozente) angewandt.

Bei der Herstellung der pharmazeutischen Lackformulierungen müssen in Bezug auf die Wahl des Penetrationsbeschleunigers, des filmbildenden Agenz und des Lösungsmittels verschiedene Faktoren beachtet werden. Die bevorzugten Zusammensetzungen müssen einen festen Film bilden können, in dem die Konzentration des pharmazeutischen Wirkstoffs und des Penetrationsbeschleunigers relativ hoch sind an der Grenzfläche zwischen dem festen Film und der Oberfläche, z. B. dem Nagel, auf der der Film gebildet wird.

Es sei angemerkt, dass der Begriff "Fugazität" verwendet wird, um anzugeben, wie hoch das Maß ist, mit dem ein gelöster Stoff aus seiner Lösung wandert und dass die Fugazität eines gelösten Stoffes dem Gesetz von Henry für den idealen Zustand folgt.

Verschiedene Methoden können angewandt werden, um die Fugazität des pharmazeutischen Wirkstoffs und des Penetrationsbeschleunigers zu erhöhen. Diese Methoden erlauben es, die Konzentration von beiden Inhaltsstoffen an der Grenzfläche zu erhöhen.

Bevorzugt können z. B. in Bezug auf die gemäß der vorliegenden Erfindung eingesetzten, antimykotisch wirksamen substituierten 2-Aminothiazole, die einen basischen Charakter aufweisen, polymere filmbildende Agenzien eingesetzt werden, die ebenfalls basisch sind. Solche basischen Verbindungen tendieren dazu, sich gegenseitig aufgrund der Ladungsverteilungen bzw. Polaritäten abzustoßen. Somit wird die Fugazität des pharmazeutischen Wirkstoffs aus der Gruppe der substituierten 2-Aminothiazole erhöht. Beispiele von polymeren filmbildenden Agenzien mit basischen Funktionalitäten oder basischen Strukturelementen sind z. B. Acrylat Copolymere mit Dimethylamino-Funktionalitäten.

Ein anderes Verfahren zur Erhöhung der Fugazität des pharmazeutischen Erzeugnisses aus der Klasse der substituierten 2-Aminothiazole besteht darin, der Lösung eine Base, wie z. B. Triethanolamin, zuzusetzen,

Im folgenden wird eine Methode beschrieben, um die Fugazität des PenetrationsBeschleunigers zu erhöhen. Die Methode beinhaltet, dass eine pharmazeutische Formulierung hergestellt wird, in der der Penetrationsbeschleuniger in einem flüchtigen Lösungsmittel gelöst ist. Das Lösungsmittel hat dabei einen Dampfdruck, der es ermöglicht, dass es innerhalb von 5 Minuten innerhalb der Anwendung verdampft ist. Weiterhin enthält die pharmazeutische Formulierung ein weniger gut flüchtiges Lösungsmittel als Cosolvenz, in dem der Penetrationsbeschleuniger nur eine begrenzte Löslichkeit von max. 5 % (Gewichtsprozent) hat. Dies bedeutet, dass das Lösungsmittel in Bezug auf das Cosolvenz schneller verdampft und das Cosolvenz etwas länger in der Formulierung verbleibt. In einem solchen Fall steigt die Fugazität des Penetrationsbeschleunigers. Ein einsetzbares Cosolvenz ist bevorzugt Propylenglykol.

Eine weitere beispielhafte Methode, um die Fugazität von sowohl der pharmazeutischen Verbindung aus der Verbindungsklasse der substituierten 2-Aminothiazole, die eine geringe Löslichkeit in Wasser haben, als auch eines Penetrationsbeschleunigers, der eine in Relation höhere Wasserlöslichkeit als die substituierten 2-Aminothiazole hat, zu erhöhen, besteht darin, in die Formulierung eine Menge von bis zu 20 % (Gewichtsprozent), bevorzugt von 1 % bis 10 % (Gewichtsprozent), besonders bevorzugt von 3 % bis 7 % (Gewichtsprozent) Wasser zuzusetzen.

Die vorausgegangenen Beschreibungen stellen exemplarische Beispiele von Methoden dar, die angewandt werden können, um die Abgabe des pharmazeutischen Wirkstoffs aus der Verbindungsklasse der substituierten 2-Aminothiazole und der Penetrationsbeschleuniger zu optimieren.

Einer oder mehrere Weichmacher können in der erfindungsgemäßen pharmazeutischen Formulierung eingesetzt werden, um die gewünschten Eigenschaften des polymeren Films, der gebildet werden soll, zu gewährleisten. Bei der Auswahl des Weichmachers und der eingesetzten Menge sollte beachtet werden, ob der Penetrationsbeschleuniger, der in der Formulierung verwendet wird, selbst weichmachende Eigenschaften besitzt. Beispiele für Weichmacher sind Propylenglycol, Diethylenglycolmonoethylether, Propylenglycolmonopropylether, Polyethylen und Poly(propylen-glycol), Triacetin, Tripropionin, Castoröl, Kampher, Phthalate, insbesondere Dibutylphthalat und Diethylphthalat, Benzylalkohol, Phenethylalkohol und N-Methyl-2-pyrrolidon und eine Mischung von zweien oder mehreren der erwähnten Weichmacher.

Gemäß dem Stand der Technik sollte der Weichmacher zu dem polymeren filmbildenden Agenz passen, das in der pharmazeutischen Formulierung eingesetzt wird.

Bevorzugt werden in der pharmazeutischen Formulierung der vorliegenden Erfindung Propylenglycol, Diethylenglycolmonoethylether, Polyethylen und Poly(propylen-glycol), 1,2,3-Propanetrioltriacetat (Triacetin) und Tripropionin und eine Mischung von zwei oder mehreren der o. a. Weichmachern eingesetzt. Propylenglycol gehört zu den bevorzugten Weichmachern.

Der Weichmacher wird in einer Menge eingesetzt, um die gewünschten Eigenschaften des polymeren Films, der durch die pharmazeutische Formulierung der vorliegenden Erfindung gebildet wird, zu erreichen. In der Regel beträgt die Menge an Weichmacher 1 % bis 25 % (Gewichtsprozent), bevorzugt 1 % bis 10 % (Gewichtsprozent).

Die pharmazeutische Formulierung kann weiterhin andere Komponenten, die dem Stand der Technik entsprechen, in Mengen, die der Fachmann kennt, enthalten. Dazu gehören farbgebende Agenzien, wie z. B. ein Farbstoff, ein färbendes Pigment, Farbplättchen, Glanzfarbstoffe oder Pigmente, wie z. B. Titandioxid und andere. Weitere Komponenten umfassen Kolloid-Stabilisatoren, UV-Stabilisatoren, antibakterielle oder bakteriostatische Verbindungen, wie z. B. antimikrobielle quartäre Ammonium-Agenzien, z. B. Cetylpyridiniumchlorid und Benzalkoniumchloride, Antioxidanzien, z. B. BHA, BHT, Parabene, Vitamin E und seine Derivative, antimikrobielle chelatbildende Agenzien, z. B. EDTA und Zitronensäure und neutralisierende Agenzien, z. B. Triethylamin, Triethanlolamin, 2-Methyl-2-amino-1-propanol, Zitronensäure und Sorbinsäure.

Die pharmazeutischen Formulierungen der vorliegenden Erfindung können auf die Membran oder den Nagel oder die Membran, also z.B. die Haut, nach jedem Verfahren aufgetragen werden, mit dem man einen Film erzeugen kann. Der Film kann durch mehrfaches Auftragen der pharmazeutischen Formulierung gebildet werden. Eine oder mehrere Schichten können jeweils nach Trocknen der voraufgegangenen Beschichtung aufgetragen werden. Periodische Erneuerung des Film ist ggf. notwendig, um die erwünschte Wirkstoffdosis dauerhaft zur Verfügung zu stellen.

Eines der wichtigsten Merkmale der pharmazeutischen Formulierung der vorliegenden Erfindung ist, dass alle flüchtigen und nicht-flüchtigen Inhaltsstoffe mit allen anderen kompatibel ist und sich Lösungen bilden können, die über einen weiten Temperaturbereich oberhalb und unterhalb von Raumtemperatur, wie z. B. von Temperaturen in einem Temperaturbereich von -15 bis +100 °C stabil sind, d.h. es dürfen in diesem Temperaturbereich keine Phasentrennungen auftreten.

Ein weiteres Charakteristikum der vorliegenden Erfindung ist es, dass die aufgetragenen Filme nach Verdampfen der Lösungsmittel und anderer flüchtigen Bestandteile stark haftende Eigenschaften haben und wasserfest sind, d. h. dass sie insbesondere bei wiederholten normalen Waschvorgängen mit Seifenwasser für mindestens einen Tag, in der Regel aber bis zu fünf oder mehr Tagen, beständig sind, also haften bleiben.

Der Gegenstand der vorliegenden Erfindung bedeutet - wie bereits erwähnt - einen wesentlichen Fortschritt gegenüber dem Stand der Technik, da mit der erfindungsgemäßen Nagellackformulierung Onychomycosen oder andere Pilzinfektionen sowie bakterielle Infektionen und Entzündungen, einschließlich Psoriasis, behandelt werden können.

Die in der vorliegenden Erfindung beschriebenen Nagellackformulierungen können nicht nur zur Therapie von Erkrankungen, sondern auch zur Vorbeugung bei Gesunden angewandt werden, wenn für diese das Risiko besteht, einer mykotischen oder anderen Infektion ausgesetzt zu sein oder zu werden.

### Beispiele

Durch die im folgenden angegebenen Beispiele 1 bis 7 werden einige für die vorliegende Erfindung typische pharmazeutische Formulierungen beschrieben, die als antimykotisch wirksames Mittel substituierte 2-Aminothiazole enthalten. Die Konzentrationsangaben der Inhaltsstoffe werden in Gewichtsprozenten in Relation zum Gesamtgewicht der pharmazeutischen Formulierung angegeben.

Die polymeren filmbildenden Agenzien werden in Pulverform oder als Pellets unter mechanischem Rühren bei Raumtemperatur in dem Lösungsmittel oder den Lösungsmittelgemischen gelöst. Nach Herstellung der Lösung wird jeweils unter Rühren das substituierte 2-Aminothiazol und der Penetrationsbeschleuniger zugegeben. Nachdem alles in Lösung gegangen ist, wird der Weichmacher und ggf. Wasser zugegeben. Die gesamte Mischung wird dann bis zur Homogenität gerührt.

### Beispiel Nr. 1

| | Gew. % |
|---|---|
| Abafungin | 2 % |
| Eudragit RL® 100 Pulver | 15 % |
| Oxacyclohexadecan-2-on (Firmenich) (Penetrationsbeschleuniger) | 15 % |
| Propylenglycol | 5 % |
| Ethanol | 60 % |
| Wasser | 3 % |

Acht Patienten mit Onychomykosen von Zehnägeln wurden jeweils einmal täglich morgens behandelt mit etwa 20 - 35 mg der Nagellackformulierung von Beispiel Nr. 1. Die Behandlung wurde 7 Tage lang täglich wiederholt. Am siebten Tag wurde der Nagellack eines jeden Anwendungszyklus durch Ablösen des Lackes mit Isopropylalkohol entfernt. Anschließend wurde ein solcher Anwendungszyklus 100 Tage lang wiederholt. Nach 45 Tagen wurden in jedem Falle wesentliche Verbesserungen des Krankheitsbildes erzielt Komplette Heilungen wurden in allen Fällen innerhalb von 100 bis 150 Tagen nach Beginn der Behandlung erzielt.

### Beispiel Nr. 2

| | Gew. % |
|---|---|
| Abafungin | 1,5 % |
| Eudragit RL® PO Pellets | 15 % |
| Oxacyclohexadecan-2-on (Firmenich) (Penetrationbeschleuniger) | 15 % |
| Propylenglycol | 5 % |
| Ethanol | 60,5 % . |
| Wasser | 3 % |

### Beispiel Nr. 3

| | Gew. % |
|---|---|
| Abafungin | 4 % |
| Eudragit RL® 100 Pulver | 15 % |
| Oxacyclohexadecan-2-on (Firmenich) (PenetrationbBeschleuniger) | 15 % |
| Propylenglycol | 5 % |
| Ethanol | 58 % |
| Wasser | 3 % |

### Beispiel Nr. 4

| | Gew. % |
|---|---|
| Abafungin | 4 % |
| Propylenglycol | 5 % |
| Hyaluronatlyase (2500 IU/cm³) | 1,3% |
| Natriumedetat | 0,1 % |
| Trometamol | 0,25 % |
| Polyacrysäure | 0,5 % |
| Kaliumsorbat | 0,1 % |
| Wasser | ad 100,00 % |

### Beispiel Nr. 5

| | Gew. % |
|---|---|
| Abafungin | 2 % |
| Hyluronatlyase (2500 IU/cm³) | 1,3 % |
| non-ionische hydrophile Salbe (gemäß DAB) | ad 100 % |

### Beispiele Nr. 6

| | Gew.% |
|---|---|
| Abafungin | 5 % |
| Pentadecalacton | 10 % |
| Eudragit RL® 100 Pulver | 25 % |
| Isopropanol | 60 % |

### Beispiel Nr. 7

| | Gew. % |
|---|---|
| Abafungin | 3 % |
| Pentadecalacton | 15 % |
| Ethanol | 82 % |

## Patentansprüche

1. Pharmazeutische, antimykotisch wirksame Nagellackformulierung zur Behandlung oder Vorbeugung von Pilzinfektionen von Nägeln und der Haut, die ein gegebenenfalls substituiertes 2-Aminothiazol der allgemeinen Formel (I) enthält 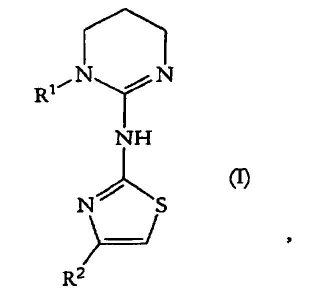 in der
R¹ Wasserstoff oder Alkyl bedeutet, und
R² einen Substituenten der Formel 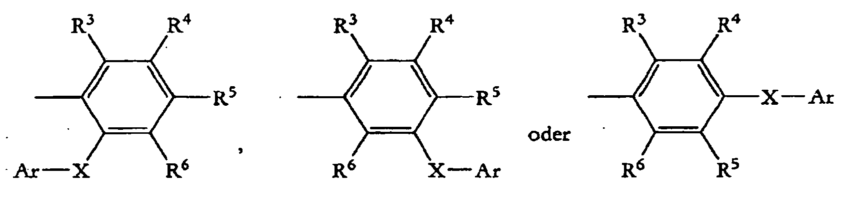 bedeutet, in dem
R³, R⁴, R⁵ und R⁶ unabhängig voneinander Wasserstoff, Halogen, Nitro, Alkyl, Alkoxy, Alkoxycarbonyl, Dialkylamino, Alkylthio, Alkylsulphinyl, Alkylsulphonyl, Halogenalkyl, Halogenalkoxy, Halogenalkylthio, Halogenalkylsulphinyl oder Halogenalkylsulphonyl bedeuten,
X Sauerstoff, Schwefel, Sulphinyl oder Sulphonyl bedeutet, und
Ar einen ggf. substituierten Arylrest bedeutet, und
deren physiologisch verträgliche Säureadditionssalze,
wobei die Verbindungen der Formel (I) im Gleichgewicht mit den tautomeren Verbindungen der Formeln (Ia) und (Ib) stehen 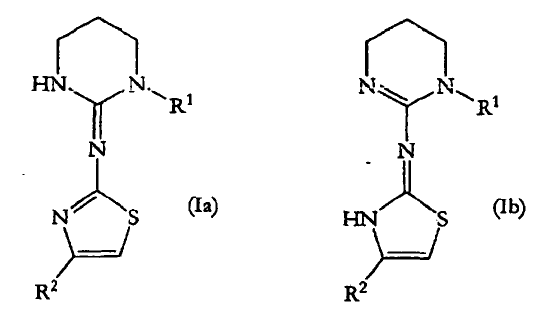 in denen R¹ and R² in jedem Fall die oben angegebenen Bedeutungen haben.

2. Pharmazeutische, antimykotisch wirksame Nagellackformulierung gemäß Anspruch 1, wobei X Sauerstoff bedeutet.

3. Pharmazeutische, antimykotisch wirksame Nagellackformulierung gemäß einem der Ansprüche 1 oder 2, die einen Penetrationsbeschleuniger enthält.

4. Pharmazeutische, antimykotisch wirksame Nagellackformulierung gemäß Anspruch 3, in der der Penetrationsbeschleuniger Hyaluronatlyase ist.

5. Pharmazeutische, antimykotisch wirksame Nagellackformulierung gemäß einem der Ansprüche 1 bis 4, die ein polymeres, filmbildendes Agenz enthält.

6. Pharmazeutische, antimykotisch wirksame Nagellackformulierung gemäß Anspruch 5, in der das filmbildende Agenz zu der Verbindungsgruppe wasserunlöslicher Polymere der Acrylatpolymere oder der Methacrylatpolymere gehört und ein Copolymer enthält, das zu der Verbindungsklasse der Alkylvinylether, Maleinsäureanhydrid, alkylierten Poly(vinylpyrrolidone) und Ammoniummethacrylate gehört.

7. Pharmazeutische, antimykotisch wirksame Nagellackformulierung gemäß einem der Ansprüche 1 bis 6, die einen Weichmacher enthält.

8. Pharmazeutische, antimykotisch wirksame Nagellackformulierung gemäß einem der Ansprüche 1 bis 7, die mindestens ein Lösungsmittel enthält.

9. Pharmazeutische, antimykotisch wirksame Nagellackformulierung gemäß Anspruch 8, die als Lösungsmittel Ethanol, Ethylacetat, Butylacetat, Isopropanol, Aceton, Methylethylketon, Triacetin, Tripropionin, Diethylenglycolmonoethylether, Isopropylacetat, oder ein Gemisch zweier oder mehrerer der aufgeführten Lösungsmittel enthält.

10. Pharmazeutische, antimykotisch wirksame Nagellackformulierung gemäß einem der Ansprüche 1 bis 9, die
zwischen 0,1 und 20 % des antimykotischen Wirkstoffes,
zwischen 0,5 und 35 % eines Penetrationsbeschleunigers,
zwischen 0,5 und 40 % eines filmbildenden Polymeren,
zwischen 5 und 80 % eines flüchtigen Lösungsmittels, und
zwischen 1,0% und 10% eines Weichmachers enthält.

11. Pharmazeutische, antimykotisch wirksame Nagellackformulierung gemäß einem der Ansprüche 1 bis 10, in der die antimykotisch wirksame Verbindung Abafungin ist.

12. Pharmazeutische, antimykotisch wirksame Nagellackformulierung gemäß einem der Ansprüche 1 bis 11, die geeignet ist, zur Behandlung von Pilzinfektionen auf infizierte Nägel aufgetragen zu werden.

13. Pharmazeutische, antimykotisch wirksame Nagellackformulierung gemäß einem der Ansprüche 1 bis 11, die geeignet ist, zur Vorbeugung von Pilzinfektionen auf Nägel aufgetragen zu werden von Personen, die der Gefahr ausgesetzt sind, sich eine Nagelpilzinfektion zuzuziehen.

## Claims

1. Pharmaceutical, antimycotically active nail lacquer formulation for the treatment or prevention of fungal infections of the nails and skin, which formulation contains an optionally substituted 2-aminothiazole of the general formula (I) 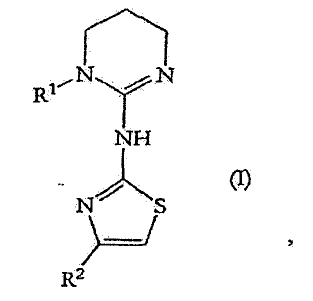 in which
R¹ represents hydrogen or alkyl and
R² represents a substituent of formula 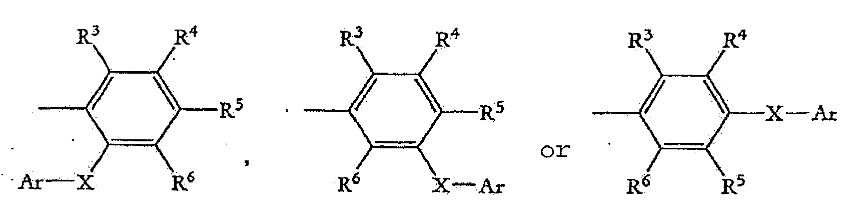 in which
R³, R⁴, R⁵ and R⁶, independently of one another, represent hydrogen, halogen, nitro, alkyl, alkoxy, alkoxycarbonyl, dialkylamino, alkylthio, alkylsulfinyl, alkylsulfonyl, haloalkyl, haloalkoxy, haloalkylthio, haloalkylsulfinyl or haloalkylsulfonyl, X represents oxygen, sulfur, sulfinyl or sulfonyl, and
Ar represents an optionally substituted aryl radical, and
the physiologically acceptable acid addition salts thereof,
wherein the compounds of formula (I) are in equilibrium with the tautomeric compounds of formulae (Ia) and (Ib) 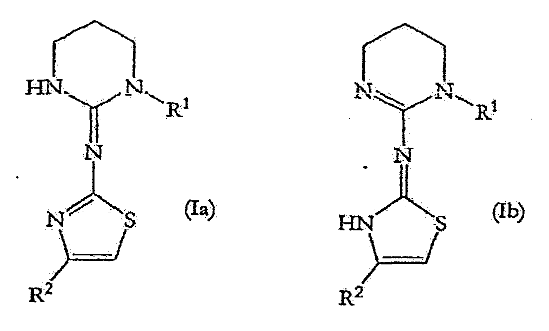 in which R¹ and R² in each case have the meanings given above.

2. Pharmaceutical, antimycotically active nail lacquer formulation according to claim 1, wherein X represents oxygen.

3. Pharmaceutical, antimycotically active nail lacquer formulation according to either claim 1 or claim 2, which contains a penetration accelerator.

4. Pharmaceutical, antimycotically active nail lacquer formulation according to claim 3, in which the penetration accelerator is hyaluronate lyase.

5. Pharmaceutical, antimycotically active nail lacquer formulation according to any one of claims 1 to 4, which contains a polymeric film-forming agent.

6. Pharmaceutical, antimycotically active nail lacquer formulation according to claim 5, in which the film-forming agent belongs to the group of water-insoluble polymers of the acrylate polymers or methacrylate polymers and contains a copolymer belonging to the class of alkyl vinyl ethers, maleic anhydride, alkylated poly(vinylpyrrolidones) and ammonium methacrylates.

7. Pharmaceutical, antimycotically active nail lacquer formulation according to any one of claims 1 to 6, which contains a plasticiser.

8. Pharmaceutical, antimycotically active nail lacquer formulation according to any one of claims 1 to 7, which contains at least one solvent.

9. Pharmaceutical, antimycotically active nail lacquer formulation according to claim 8, which contains as solvent ethanol, ethyl acetate, butyl acetate, isopropanol, acetone, methyl ethyl ketone, triacetin, tripropionin, diethylene glycol monoethyl ether, isopropyl acetate, or a mixture of two or more of the mentioned solvents.

10. Pharmaceutical, antimycotically active nail lacquer formulation according to any one of claims 1 to 9, which contains
from 0.1 to 20 % of the antimycotic active ingredient,
from 0.5 to 35 % of a penetration accelerator,
from 0.5 to 40 % of a film-forming polymer,
from 5 to 80 % of a volatile solvent and
from 1.0 to 10 % of a plasticiser.

11. Pharmaceutical, antimycotically active nail lacquer formulation according to any one of claims 1 to 10, in which the antimycotically active compound is abafungin.

12. Pharmaceutical, antimycotically active nail lacquer formulation according to any one of claims 1 to 11, which is suitable for application to infected nails in order to treat fungal infections.

13. Pharmaceutical, antimycotically active nail lacquer formulation according to any one of claims 1 to 11, which is suitable for application to the nails of persons who are at risk of a nail fungal infection in order to prevent fungal infections.

## Revendications

1. Formulation de vernis à ongles pharmaceutique ayant une activité antimycosique, destinée au traitement ou à la prévention d'infections fongiques des ongles et de la peau et contenant un 2-aminothiazole le cas échéant substitué de formule générale (1) 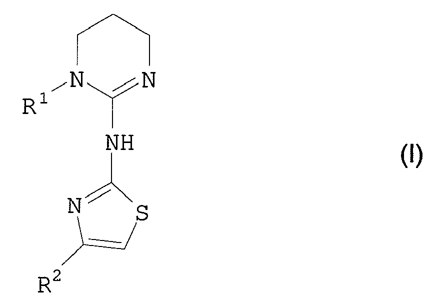 dans laquelle
R¹ représente l'hydrogène ou un alkyle, et
R² représente un substituant de formule 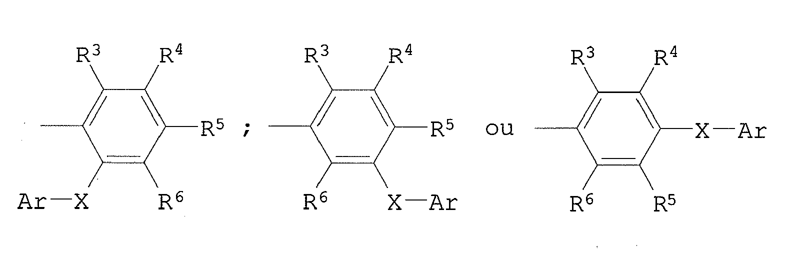 où
R³, R⁴, R⁵ et R⁶ représentent indépendamment les uns des autres l'hydrogène, un halogène, un groupe nitro, alkyle, alcoxy, alcoxycarbonyle, dialkylamino, alkylthio, alkylsulfinyle, alkylsulfonyle, haloalkyle, haloalcoxy, haloalkylthio, haloalkylsulfinyle ou haloalkylsulfonyle,
X représente l'oxygène, le soufre, un sulfinyle ou un sulfonyle, et
Ar représente un résidu aryle le cas échéant substitué,
ainsi que leurs sels d'addition acide physiologiquement acceptables,
les composés de la formule (I) se trouvant à l'équilibre avec les composés tautomères des formules (la) et (Ib) 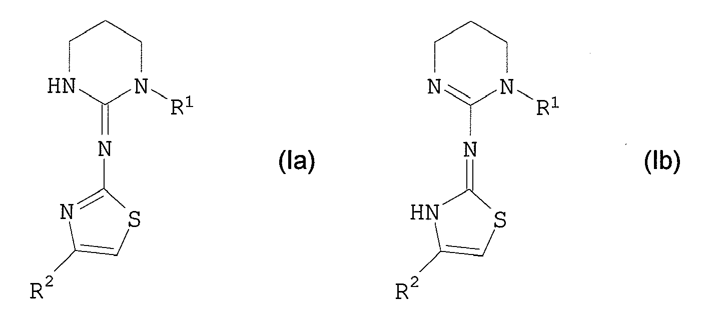 où R¹ et R² ont dans tous les cas les significations indiquées ci-dessus.

2. Formulation de vernis à ongles pharmaceutique ayant une activité antimycosique selon la revendication 1, dans laquelle X représente l'oxygène.

3. Formulation de vernis à ongles pharmaceutique ayant une activité antimycosique selon l'une des revendications 1 ou 2, qui contient un accélérateur de pénétration.

4. Formulation de vernis à ongles pharmaceutique ayant une activité antimycosique selon la revendication 3, dans laquelle l'accélérateur de pénétration est la hyaluronate lyase.

5. Formulation de vernis à ongles pharmaceutique ayant une activité antimycosique selon l'une des revendications 1 à 4, qui contient un agent filmogène polymère.

6. Formulation de vernis à ongles pharmaceutique ayant une activité antimycosique selon la revendication 5, dans laquelle l'agent filmogène appartient au groupe de composés comprenant des polymères insolubles dans l'eau, des polymères d'acrylate ou des polymères de méthacryle et contient un copolymère appartenant à la classe de composés comprenant des éthers vinyliques d'alkyles, l'anhydride maléique, des polyvinylpyrrolidones alkylées et des méthacrylates d'ammonium.

7. Formulation de vernis à ongles pharmaceutique ayant une activité antimycosique selon l'une des revendications 1 à 6, qui contient un plastifiant.

8. Formulation de vernis à ongles pharmaceutique ayant une activité antimycosique selon l'une des revendications 1 à 7, qui contient au moins un solvant.

9. Formulation de vernis à ongles pharmaceutique ayant une activité antimycosique selon la revendication 8, qui contient en tant que solvant de l'éthanol, de l'acétate d'éthyle, de l'acétate de butyle, de l'isopropanol, de l'acétone, de la méthyléthylcétone, de la triacétine, de la tripropionine, du diéthylène glycol monoéthyléther, de l'acétate d'isopropyle ou un mélange de deux ou plusieurs des solvants énumérés.

10. Formulation de vernis à ongles pharmaceutique ayant une activité antimycosique selon l'une des revendications 1 à 9, qui contient
de 0,1 à 20 % du principe actif antimycosique,
de 0,5 à 35 % d'un accélérateur de pénétration,
de 0,5 à 40 % d'un polymère filmogène,
de 5 à 80 % d'un solvant volatil, et
de 1,0% à 10% d'un plastifiant.

11. Formulation de vernis à ongles pharmaceutique ayant une activité antimycosique selon l'une des revendications 1 à 10, dans laquelle le composé ayant une activité antimycosique est l'abafungine.

12. Formulation de vernis à ongles pharmaceutique ayant une activité antimycosique selon l'une des revendications 1 à 11, qui est appropriée pour être appliquée sur des ongles infectés afin de traiter des infections fongiques.

13. Formulation de vernis à ongles pharmaceutique ayant une activité antimycosique selon l'une des revendications 1 à 11, qui est appropriée pour être appliquée, dans le but de prévenir des infections fongiques, sur les ongles de personnes exposées à un risque de contracter une infection fongique des ongles.
